(19) Europäisches Patentamt
European Patent Office
Office européen des brevets

(11) Veröffentlichungsnummer: **0 468 308 A2**

(12) **EUROPÄISCHE PATENTANMELDUNG**

(21) Anmeldenummer: **91111635.8**

(51) Int. Cl.5: **C07C 31/38**, C07C 29/10

(22) Anmeldetag: **12.07.91**

(30) Priorität: **25.07.90 DE 4023625**

(43) Veröffentlichungstag der Anmeldung: **29.01.92 Patentblatt 92/05**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Böhm, Stefan, Dr.**
**Carl-Leverkus-Strasse 30**
**W-5090 Leverkusen 1(DE)**
Erfinder: **Marhold, Albrecht, Dr.**
**Carl-Duisberg-Strasse 329**
**W-5090 Leverkusen 1(DE)**
Erfinder: **Bielefeldt, Dietmar, Dr.**
**Beuthener Strasse 13**
**W-4030 Ratingen 6(DE)**

(54) **Verfahren zur Herstellung von Beta-Fluoralkoholen.**

(57) Verfahren zur Herstellung von β-Fluoralkoholen der Formel (I)

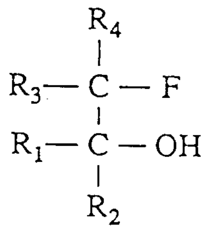

(I)

in welcher
$R^1$ und $R^2$ für geradkettiges oder verzweigtes Alkyl stehen und
$R^3$ und $R^4$ für Wasserstoff oder Alkyl stehen,
wobei man Epoxide der Formel (II)

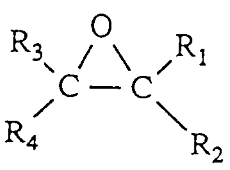

(II)

mit Natrium- oder Kaliumhydrogenfluorid unter Druck in Gegenwart von Verdünnungsmitteln umsetzt.

EP 0 468 308 A2

Die Erfindung betrifft ein neues Verfahren zur Herstellung von β-Fluoralkoholen, die wertvolle Zwischenprodukte für Synthesen in der organischen Chemie sind. Sie können z.B. als Vorprodukte zur Herstellung von Herbiziden eingesetzt werden.

Es ist bereits bekannt, daß die ringöffnende Fluorierung von Epoxiden mit Fluorierungsmitteln zu β-Fluoralkoholen führt (vgl. z.B. J. Chem. Soc. Chem. Comm. 1989, (1848)). Nachteilig an diesem Verfahren ist, daß je nach Fluorierungsmethode unterschiedliche Stereo- und Regioselektivitäten gefunden werden. Die Ausbeuten sind aufgrund von Nebenreaktionen (z.B. Polymerisation) meist unbefriedigend (J. Chem. Soc. C, 1968, 2129).

Bisher wurden im Falle der einfachen aliphatischen Epoxide nur Fluorierungsmethoden angewendet, die bei niedrigen Temperaturen arbeiten, wie z.B Fluorwasserstoff in bestimmten Lösungsmitteln (J. Gew. Chem. (USSR), 19, 95(1949)), HF/Pyridin-Komplexe (Isr. J. Chem. 17, 148 (1978) oder HF/Amin-Komplexe (J. Org. Chem. 53, 1026(1988)). Nachteilig an diesen Methoden ist, daß man im Falle unsymmetrischer Epoxide Produkte oder Produktgemische erhält, bei denen das Fluoratom überwiegend oder ausschließlich an das höher substituierte C-Atom gebunden vorliegt.

Es ist daher im allgemeinen mit diesen Methoden nicht oder nur sehr schlecht möglich, Fluoralkohole herzustellen, bei denen das Fluoratom an dem geringer substituierten C-Atom gebunden vorliegt.

Es ist weiterhin bekannt, daß Ethylenoxid mit Bifluoriden zu Ethylenfluorhydrin umgesetzt werden kann (J. Gew. Chem. (USSR), 19, 95 (1949)). Nachteilig an diesem Verfahren sind die geringen Ausbeuten. Da Ethylenoxid eine symmetrisch substituierte Verbindung ist, spielt die Frage der Regioselektivität keine Rolle.

Es wurde nun gefunden, daß man β-Fluoralkohole der Formel (I)

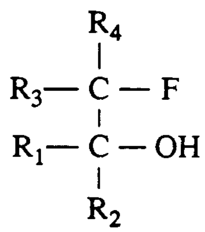

(I)

in welcher

$R^1$ und $R^2$ für geradkettiges oder verzweigtes Alkyl stehen und

$R^3$ und $R^4$ unabhängig voneinander für Wasserstoff oder geradkettiges oder verzweigtes Alkyl stehen,

erhält, wenn man

Epoxide der Formel (II)

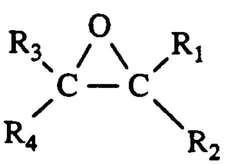

(II)

in welcher

$R^1$, $R^2$, $R^3$ und $R^4$ die oben angegebene Bedeutung haben,

mit Kaliumhydrogenfluorid ($KHF_2$) oder Natriumhydrogenfluorid ($NaHF_2$) unter Druck in Gegenwart von Verdünnungsmitteln umsetzt.

Es ist als überraschend zu bezeichnen, daß man unter diesen drastischen Bedingungen die Fluoralkohole in so guten Ausbeuten erhält, da bekannt ist, daß Epoxide zu ausbeutemindernden Nebenraktionen neigen (J. Chem. Soc. C, 1968, 2129).

Außerdem ist es überraschend, daß man eine ausgeprägte Regioselektivität der Reaktion beobachtet, die den bisher beobachteten Regioselektivitäten bei der Fluorierung einfacher aliphatischer Epoxide entgegengesetzt ist (Bull. Soc. Chim. France (1968) 2929). So ist z.B. nach dem neuen Verfahren möglich, Fluor-tert.-butanol als Hauptprodukt bei der Umsetzung von Isobutylenoxid mit $KHF_2$ zu erhalten, während man bei den üblichen Verfahren einen isomeren Alkohol als Hauptprodukt oder einziges Produkt erhält.

Das Verfahren zeichnet sich durch eine Reihe von Vorteilen gegenüber anderen Methoden aus. Es ist sehr einfach und mit billigen Ausgangsmaterialien und Agenzien durchzuführen. Außerdem erhält man durch die geänderte Regioselektivität Zugang zu Verbindungen, die sonst nur schlecht dargestellt werden können.

Mit Hilfe des erfindungsgemäßen Verfahrens erhält man vorzugsweise β-Fluoralkohole der Formel (I), in welcher $R^1$ und $R^2$ unabhängig voneinander für geradkettiges oder verzweigtes Alkyl mit 1 bis 4 Kohlenstoffatomen, besonders bevorzugt für Methyl, Ethyl, n- oder iso-Propyl, insbesondere für Methyl oder Ethyl stehen, $R^3$ und $R^4$ unabhängig voneinander für Wasserstoff, geradkettiges oder verzweigtes Alkyl mit 1 bis 4 Kohlenstoffatomen, besonders bevorzugt für Wasserstoff, Methyl oder Ethyl und insbesondere für Wasserstoff stehen.

Verwendet man beispielsweise Isobutylenoxid als Ausgangsverbindung, so kann das erfindungsgemäße Verfahren durch folgendes Formelschema veranschaulicht werden:

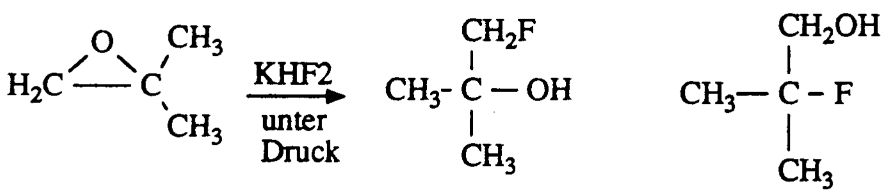


75 : 25

Die zur Durchführung des erfindungsgemäßen Verfahrens benötigten Epoxide der Formel (II) sind bekannt.

Zur Durchführung des erfindungsgemäßen Verfahrens setzt man im allgemeinen äquivalente Mengen an Epoxid der Formel (II) und an Fluorierungsmittel um oder man setzt das Fluorierungsmittel im Überschuß ein.

Bevorzugt wird das Natrium- bzw. Kaliumhydrogenfluorid im Überschuß in einer Menge von 1,01 bis 1,5 Äquivalenten, besonders bevorzugt in einer Menge von 1,05 bis 1,3 Äquivalenten eingesetzt.

Die Reaktionstemperaturen können bei der Durchführung des erfindungsgemäßen Verfahren in einem größeren Bereich variiert werden. Im allgemeinen arbeitet man bei Temperaturen zwischen 80 °C bis 300 °C, vorzugsweise zwischen 110 °C und 180 °C.

Das erfindungsgemäße Verfahren wird in geschlossenen Apparaturen unter Druck durchgeführt. Der Druck kann durch Aufdrücken eines Inertgases in weiten Grenzen variiert werden. Vorzugsweise wird bei Drucken von 1 bis 20 bar gearbeitet.

Die erfindungsgemäße Umsetzung kann sowohl kontinuierlich als auch diskontinuierlich durchgeführt werden.

Im allgemeinen ist es vorteilhaft, das erfindungsgemäße Verfahren in Gegenwart eines Lösungsmittels durchzuführen.

Geeignete Lösungsmittel sind solche, die unter den Reaktionsbedingungen inert oder weitgehend inert sind. In Frage kommen z.B. Ether z.B. Ethylpropylether, n-Butylether, Di-n-butylether, Diisobutylether, Diisoamylether, Diisopropylether, Anisol, Phenetol, Cyclohexylmethylether, Diethylether, Tetrahydrofuran, Dioxan, Thioanisol, β,β'-Dichlordiethylether; Polyethylenglykolether wie Ethylglykoldimethylether, -diethylether, di-n-propylether, -diisopropylether, -di-n-butylether, -diisobutylether, -di-sek.-butylether, di-tert.-butylether, Diethylenglykoldimethylether, -diethylether, -di-n-propylether, -diisopropylether, -di-sek.-butylether, -di-tert.-butylether; analoge Diether des Triethylenglykols und des Tetraethylenglykols; Diethylenglykolmonomethylether, Diethylenglykolmonoethylether, Triethylenglykolmonobutylether, Ethylenglykol, Diethylenglykol, Triethylenglykol, Tetraethylenglykol, 1,2-Propandiol, 1,3-Propandiol; Sulfoxide wie Dimethylsulfoxid, Diethylsulfoxid, Dimethylsulfon, Diethylsulfon, Methylethylsulfin, Tetramethylensulfon (Sulfolan); N-Methylpyrrolidon, Dimethylformamid, Hexamethylphosphorsäuretriamid, N,N'-Dimethylpropylenharnstoff, N-Methyl-caprolactam; Benzol, Toluol; Acetonitril und hinsichtlich Qualität und Quantität beliebige Gemische dieser Lösungsmittel.

Bevorzugte Lösungsmittel sind Diethylenglykol, Triethylenglykol, Tetraethylenglykol, höhere Glykole gegebenenfalls auch im Gemisch sowie N-Methylpyrrolidon; besonders bevorzugt sind Glykole. Bezogen auf die Ausgangsverbindung der Formel (II) können beliebige Mengen Lösungsmittel verwendet werden. Geeignet sind z.B. solche Lösungsmittelmengen, daß eine 10 bis 80 gew.-%ige Mischung der Ausgangsverbindung im jeweiligen Lösungsmittel oder Lösungsmittelgemisch vorliegt. Bevorzugt beträgt die Konzentration des Epoxids in dieser Mischung 35-65 Gew.-%.

Die Reaktion kann auch in Gegenwart von Katalysatoren, beispielsweise Kronenethern durchgeführt werden. Sie haben im allgemeinen nur einen geringen Einfluß auf den Umsatz der Ausgangsverbindungen und die Ausbeuten.

Die Aufarbeitung des Reaktionsgemisches erfolgt durch Destillation, indem man bei Drücken zwischen 10 mbar und Normaldruck das Produkt oder Produktgemisch vom Lösungsmittel und den Salzen abdestilliert und gegebenenfalls durch eine erneute Destillation reinigt oder trennt.

Das erfindungsgemäße Verfahren wird durch die folgenden Beispiele veranschaulicht.

Beispiel 1

Umsetzung von Isobutylenoxid mit Kaliumhydrogenfluorid

In einem Autoklaven wird eine Mischung aus 2,7 kg (34,6 mol) Kaliumhydrogenfluorid und 3 l Tetraethylenglykol vorgelegt und auf 150 °C erhitzt. Innerhalb von 4 Stunden werden 2 kg (28,2 mol) Isobutylenoxid zugepumpt. Anschließend wird 4 Stunden bei 150 °C nachgerührt. Nach dem Abkühlen und Entspannen wird das Produkt unter vermindertem Druck aus dem Reaktionsgemisch herausdestilliert. Man erhält 1,64 kg (63 %) Rohprodukt, das laut GC und NMR-Spektren 75 % Fluortert.-butanol (1-Fluor-2-methyl-propan-2-ol) und 25 % des isomeren Fluoralkohols 2-Fluor-2-methyl-propan-1-ol enthält Durch fraktionierende Destillation kann man reines Fluor-tert.-butanol erhalten.

Beispiel 2

Umsetzung von cis-2.3-Butenoxid mit Kaliumhydrogenfluorid

Die Umsetzung erfolgt analog Beispiel 1. Man erhält in 59 % Ausbeute 3-Fluor-butan-2-ol stereochemisch einheitliche Verbindung (Racemat aus R,R- und S,S-Konfiguration).

Beispiel 3

Umsetzung von 1,2-Butenoxid mit Kaliumhydrogenfluorid

Die Umsetzung erfolgt analog Beispiel 1. Man erhält in 69 %-iger Ausbeute ein Gemisch aus 1-Fluor-butan-2-ol (70 %) und 2-Fluor-butan-1-ol (30 %).

Die nach dem erfindungsgemäßen Verfahren herstellbaren β-Fluoralkohole der Formel (I) sind wertvolle Ausgangsstoffe zur Synthese von biologisch aktiven Verbindungen, wie z.B. zur Synthese von substituierten Triazolinonen (vgl. EP-A 294 666), welche gute herbizide Eigenschaften besitzen).

So erhält man beispielsweise durch Umsetzung von β-Fluoralkoholen der Formel (I) mit Cyanwasserstoff oder Aryl- oder Alkylcyaniden, insbesondere Cyanwasserstoff, in Gegenwart von Schwefelsäure und gegebenenfalls in Gegenwart eines Verdünnungsmittels wie beispielsweise Dioxan bei Temperaturen zwischen 10 bis 120 °C, vorzugsweise 40 bis 80 °C, β-Fluor-tert.-alkylamide der Formel (III),

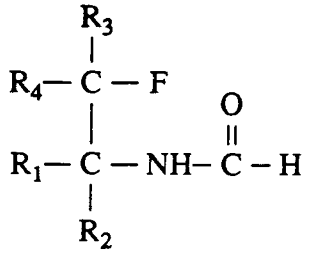

bzw.

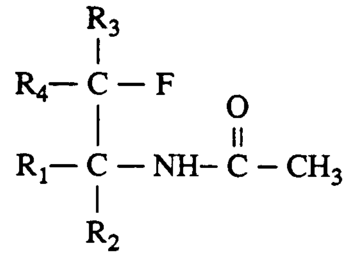

welche gegebenenfalls nach Zwischenisolierung verseift werden zu den β-Fluor-tert.-alkylamin-hydrochloriden der Formel (IV)

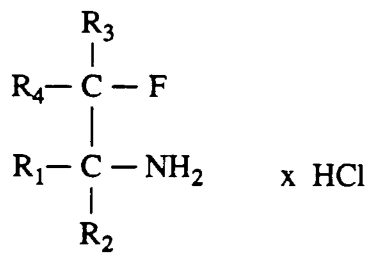

(IV).

Aus den Aminhydrochloriden der Formel (IV) bzw. den freien Aminen erhält man durch Umsetzung mit Phosgen gegebenenfalls in Geegnwart eines Verdünnungsmittels wie beispielsweise o-Dichlorbenzol gegebenenfalls in Gegenwart einer Base wie beispielsweise Triethylamin Isocyanate, welche dann gemäß den in EP-A 294 666 beschriebenen Verfahren zu herbizid wirksamen Verbindungen umgesetzt werden können.

Beispiel zur Herstellung von

$\beta$-Monofluor-tert.-butylisocyanat:

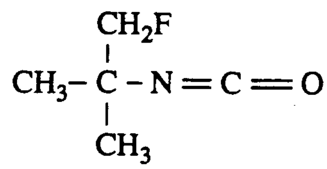

20 g (0.156 mol) N-(Fluor-tert.-butyl)- aminhydrochlorid werden in 100 ml o-Dichlorbenzol gelöst. Bei 140 °C werden 45 g (0,5 mol) Phosgen eingeleitet. Nach Austreiben des überschüssigen Phosgens mit Stickstoff wird im Vakuum fraktioniert.

Man erhält 9,5 g (52 % der Theorie) $\beta$-Monofluor-tert.-butylisocyanat vom Schmelzpunkt 110-112 °C.

N-(Fluor-tert.-butyl)aminhydrochlorid:

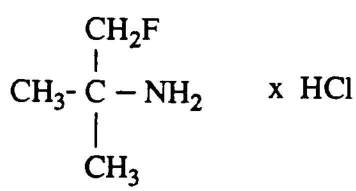

119 g (1 Mol) N-(Fluor-tert.-butyl)-formamid werden mit 1000 ml 20 %iger Salzsäure versetzt und 4 Stunden bei 70 °C gerührt. Anschließend destilliert man unter vermindertem Druck bis zur Trockne und trocknet den Rückstand im Vakuum bis zur Gewichtskonstanz.

Ausbeute: 120 g (94 % der Theorie)
Schmelzpunkt: 250 °C

N-(Fluor-tert.-butyl)-formamid:

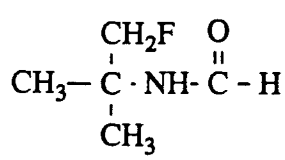

92 g (1 Mol) 1-Fluor-2-methyl-propan-2-ol (vgl. Beispiel 1 der vorliegenden Anmeldung) werden bei 10 bis 15 °C in 80 ml (2 Mol) Blausäure gelöst. Unter Rühren werden langsam 140 g (1,4 Mol) konz. Schwefelsäure zugetropft. Dann wird 15 Stunden bei Raumtemperatur nachgerührt.

Überschüssige Blausäure wird im Vakuum abgezogen, der Rückstand wird auf 250 ml Eiswasser gegeben und 5 mal mit 100 ml Dichlormethan extrahiert. Nach Trocknen mit Magnesiumsulfat wird das Lösungsmittel im Vakuum abdestilliert.

| | |
|---|---|
| Ausbeute: | 86 g (72 % der Theorie) |
| Siedepunkt: | 53-54 °C (0,05 mbar) |
| Reinheit: | 95 % (Gaschromatographie). |

## Patentansprüche

**1.** Verfahren zur Herstellung von β-Fluoralkoholen der Formel (I)

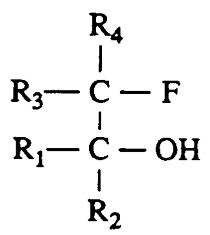

(I)

in welcher

$R^1$ und $R^2$ für geradkettiges oder verzweigtes Alkyl stehen und

$R^3$ und $R^4$ unabhängig voneinander für Wasserstoff oder geradkettiges oder verzweigtes Alkyl stehen,

wobei man Epoxide der Formel (II)

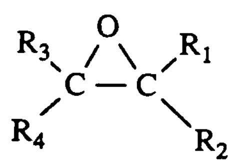

(II)

in welcher

$R^1$, $R^2$, $R^3$ und $R^4$ die oben angegebenen Bedeutungen haben,

mit Natrium- oder Kaliumhydrogenfluorid unter Druck in Gegenwart von Verdünnungsmitteln umsetzt.

**2.** Verfahren gemäß Anspruch 1, wobei $R^1$ und $R^2$ für geradkettiges oder verzweigtes Alkyl mit 1 bis 4 Kohlenstoffatomen stehen.

**3.** Verfahren gemäß Anspruch 1, wobei $R^3$ und $R^4$ unabhängig voneinander für Wasserstoff oder geradkettiges oder verzweigtes Alkyl mit 1 bis 4 Kohlenstoffatomen stehen.

**4.** Verfahren gemäß Anspruch 1, wobei $R^1$ und $R^2$ unabhängig voneinander für Methyl, Ethyl, n- oder iso-Propyl stehen.

**5.** Verfahren gemäß Anspruch 1, wobei $R^3$ und $R^4$ unabhängig voneinander für Wasserstoff, Methyl oder Ethyl stehen.

6. Verfahren gemäß Anspruch 1, wobei $R^1$ und $R^2$ für Methyl und $R^3$ und $R^4$ für Wasserstoff stehen.

7. Verfahren gemäß Anspruch 1, dadurch gekennzeichnet, daß man die Umsetzung bei Temperaturen zwischen 80 °C und 300 °C durchführt.

8. Verfahren gemäß Anspruch 1, dadurch gekennzeichnet, daß man äquivalente Mengen an Epoxid der Formel (II) und Natrium- bzw. Kaliumhydrogenfluorid oder daß man pro Äquivalent an Epoxid der Formel (II) einen Überschuß an Natrium- bzw. Kaliumhydrogenfluorid umsetzt.

9. Verfahren gemäß Anspruch 8, dadurch gekennzeichnet, daß man pro Äquivalent an Epoxid der Formel (II) 1,01 bis 1,5 Äquivalente an Natrium- bzw. Kaliumhydrogenfluorid einsetzt.